# EUROPEAN PATENT APPLICATION

(11) **EP 4 455 288 A1**
(43) Date of publication of application: **30.10.2024**
(21) Application number: 22911360.0
(22) Date of filing: 22.12.2022
(51) Int. Cl.: C12N 15/54, A61K 38/50, A61K 47/66, A61K 48/00, A61P 43/00, C07K 14/08, C07K 14/415, C07K 19/00, C12N 9/10, C12N 15/11, C12N 15/40, C12N 15/62, C12N 15/63

(54) **ENZYME, COMPLEX, RECOMBINANT VECTOR, THERAPEUTIC AGENT FOR GENETIC DISORDER, AND POLYNUCLEOTIDE**

(30) Priority: 24.12.2021 JP 2021210380
(71) Applicant: Gecort Co., Ltd., Yokohama-shi, Kanagawa 220-0011 (JP)
(72) Inventor: TSUKAHARA, Toshifumi, Nomi-shi, Ishikawa 923-1292 (JP); Ruchika, Nomi-shi, Ishikawa 923-1292 (JP)
(74) Representative: v. Bezold & Partner Patentanwälte - PartG mbB
(86) International application number: PCT/JP2022/047410
(87) International publication number: WO 2023/120658

(57) **Abstract**

An enzyme has an activity that converts uridine in RNA to cytidine. A complex includes: the enzyme; and a sequence recognition module that allows the enzyme to act specifically on uridine that has occurred in mRNA due to mutation.

## Description

### Technical Field

The present disclosure relates to an enzyme, a complex, a recombinant vector, a genetic disease therapeutic agent, and a polynucleotide, for converting uridine in RNA to cytidine.

### Background Art

A nonsense mutation is a mutation that converts a codon encoding an amino acid to a stop codon. The stop codon that has occurred due to the mutation is called premature stop codon, and translation does not proceed after the premature stop codon. Diseases caused by nonsense mutations are called nonsense mutation-type genetic diseases, and include various diseases such as cystic fibrosis (CF) and Duchenne muscular dystrophy (DMD).

When a particular compound is administered to a patient showing difficulty in the production of an original protein due to a premature stop codon, the phenomenon called read-through occurs to allow continuation of translation by skipping of the premature stop codon. Compounds that cause the read-through have been expected to enable treatment of diseases by allowing synthesis of proteins that are similar to the wild-type normal proteins, to improve nonsense mutation-type genetic diseases and treat the diseases. As a compound having such a read-through activity, Patent Literature 1 discloses an aminoglycoside compound.

There is a concern that aminoglycoside compounds may have side effects such as occurrence of deafness as a complication, and exacerbation of symptoms in patients with renal disorder. Furthermore, those compounds often cause read-through of not only the target premature stop codon, but also the original stop codon, to cause addition of an excessive C-terminal peptide to the normal protein. Thus, there is a concern that the additional peptide may cause side effects.

As a therapeutic method for genetic diseases, genome editing has been expected to be useful. Genome editing is a technique in which the double strand of genomic DNA is site-specifically cleaved, and then the DNA is repaired by non-homologous end joining or homologous recombination, to induce mutation. However, since accurate genome editing of all target cells in the body is extremely difficult at present, cells whose genome has been accurately edited need to be selected after carrying out genome editing. Further, there are a number of ethical concerns in genome editing. Although genome editing is a method suitable for ex vivo applications or fertilized eggs, its systemic application to patients is difficult.

For correction of mutation from guanosine to adenosine in a gene, Non Patent Literature 1 discloses a method that uses a deaminase domain of adenosine deaminase 1 (ADAR1), which acts on RNA, and an MS2 system including an MS2 phage-derived RNA-binding coat protein (MS2 coat protein) and an RNA (MS2 RNA) that specifically binds to the protein. In this method, when the target RNA is an RNA containing adenosine to be corrected, a guide RNA is constituted with the complementary strand of the target RNA. The MS2 RNA, the MS2 coat protein, and the ADAR1 are sequentially bound to the guide RNA, to provide an artificial enzyme complex. By the guide RNA, the ADAR1 is guided to the vicinity of the target RNA. By using a guide RNA that uses cytidine as the base corresponding to the adenosine to be corrected, deamination by the ADAR1 can be easily achieved since the adenosine mismatches with the cytidine in the guide RNA. When adenosine is converted to inosine by deamination, the inosine is read as guanosine during translation since inosine forms a base pair with cytidine.

Patent Literature 2 discloses a method in which a nucleic acid base constituting a premature stop codon is modified to cause read-through, to allow biosynthesis of the full-length protein from mRNA. This method is a technique based on the fact that read-through of a premature stop codon occurs by introduction of a functional group to a nucleic acid base constituting the premature stop codon by methylation or halogenation.

### Citation List

### Patent Literature

Patent Literature 1: Unexamined Japanese Patent Application Publication (Translation of PCT Application) No. 2009-532461
Patent Literature 2: Unexamined Japanese Patent Application Publication No. 2020-124155

### Non Patent Literature

Non Patent Literature 1: Md TA Azad and others, "Site-directed RNA editing by adenosine deaminase acting on RNA for correction of the genetic code in gene therapy", Gene Therapy, 2017, 24, 779-786

### Summary of Invention

### Technical Problem

There are only three stop codons: UAA, UAG, and UGA. By converting uridine to another base, a nonsense mutation can be converted to a codon encoding an amino acid.

In plants, RNA editing from cytidine in mRNA to uridine is known. For example, pentatricopeptide repeat (PPR) protein of *Physcomitrium patens,* which binds to RNA in a base sequence-specific manner, contains repeats of a PPR motif containing two α-helix structures of about 35 amino acids. Among PPR proteins, PPR-DYW, which has an extension (E) domain and an Asp-Tyr-Trp (DYW) domain at the C-terminus, has deaminase activity, and is known to catalyze deamination of cytidine to convert the cytidine to uridine.

However, no enzyme that converts uridine in RNA to cytidine has so far been identified. Therefore, conversion of uridine in RNA to cytidine has been impossible. If uridine that has occurred in RNA due to mutation can be converted to cytidine, mRNA that has undergone nonsense mutation can be converted such that a full-length protein can be synthesized therefrom. Furthermore, repair of a mutated mRNA having a mutation from C to T, (C > T) point mutation, in a gene becomes possible.

The present disclosure was made in view of the above circumstances, and an objective of the disclosure is to provide an enzyme, a complex, a recombinant vector, a genetic disease therapeutic agent, and a polynucleotide, capable of converting uridine that has occurred in mRNA due to mutation to cytidine.

### Solution to Problem

*Physcomitrium patens* is known to show RNA editing from uridine to cytidine. The present inventors intensively studied to identify an enzyme that catalyzes the reaction, thereby completing the present disclosure.

An enzyme according to a first aspect of the present disclosure has an activity that converts uridine in RNA to cytidine.

The enzyme according to the first aspect of the present disclosure may include:
(i) a region having the amino acid sequence of SEQ ID NO: 1 or SEQ ID NO: 2; or
(ii) a region having an amino acid sequence having a sequence identity of 90% or more to the amino acid sequence of SEQ ID NO: 1 or SEQ ID NO: 2.

A complex according to a second aspect of the present disclosure includes:
the enzyme according to the first aspect of the present disclosure; and
a sequence recognition module that allows the enzyme to act specifically on uridine that has occurred in mRNA due to mutation.

The uridine may be uridine contained in a premature stop codon formed by nonsense mutation.

The sequence recognition module may include:
a guide RNA complementary to at least part of a target RNA containing the uridine;
an RNA-binding coat protein derived from MS2 phage; and
an MS2 RNA that specifically binds to the RNA-binding coat protein, wherein
the guide RNA forms a fusion RNA with the MS2 RNA, and
the enzyme forms a fusion protein with the RNA-binding coat protein and is bound to the guide RNA through binding of the RNA-binding coat protein to the MS2 RNA.

A recombinant vector according to a third aspect of the present disclosure includes:
a fusion protein of the enzyme according to the first aspect of the present disclosure and an MS2 phage-derived RNA-binding coat protein; and
a fusion RNA of an MS2 RNA that specifically binds to the RNA-binding coat protein and a guide RNA complementary to at least part of a target RNA containing uridine that has occurred in mRNA due to mutation, wherein
the recombinant vector allows intracellular expression and formation of a complex in which the enzyme is bound to the guide RNA through binding of the RNA-binding coat protein to the MS2 RNA.

A genetic disease therapeutic agent according to a fourth aspect of the present disclosure includes:
the complex according to the second aspect of the present disclosure; or
the recombinant vector according to the third aspect of the present disclosure.

A polynucleotide according to a fifth aspect of the present disclosure encodes the enzyme according to the first aspect of the present disclosure.

A polynucleotide according to a sixth aspect of the present disclosure encodes a complex including:
a fusion protein of the enzyme according to the first aspect of the present disclosure and an MS2 phage-derived RNA-binding coat protein; and
a fusion RNA of an MS2 RNA that specifically binds to the RNA-binding coat protein and a guide RNA complementary to at least part of a target RNA containing uridine that has occurred in mRNA due to mutation.

### Advantageous Effects of Invention

By the present disclosure, uridine that has occurred in mRNA due to mutation can be converted to cytidine.

### Brief Description of Drawings

FIG. 1 is a diagram illustrating the configuration of the construct according to Example 1;
FIG. 2 is a diagram illustrating the base sequence detected in Example 1;
FIG. 3 is a diagram for illustrating the construct according to Example 2; FIG. 3A is a diagram illustrating the region included in each defective protein; FIG. 3B is a schematic diagram of an artificial RNA editing enzyme complex; and
FIG. 4 is a diagram illustrating the base sequence detected in Example 2.

### Description of Embodiments

Embodiments according to the present disclosure are described below with reference to drawings. The present disclosure is not limited by the following embodiments and drawings. In the following embodiments, expressions such as "have", "include", or "contain" also encompass the meaning of "consist of" or "be constituted by"

### Embodiment 1

An enzyme according to the present embodiment has an activity that converts uridine in RNA to cytidine. More specifically, the enzyme catalyzes the reaction of transferring an amino group to uracil, which is the nucleic acid base of uridine, to generate cytidine. The enzyme is not limited as long as the enzyme has an activity that converts uridine in RNA to cytidine.

Examples of the enzyme according to the present embodiment include aminotransferase derived from *Physcomitrium patens.* As shown in Example 2 below, for the PPR-DYW deaminase activity, the E2 (also called E+; hereinafter referred to as "E2") domain of PPR protein is indispensable. Similarly, the E2 domain is indispensable for the transamination activity by the enzyme according to the present embodiment. The E2 domain has the amino acid sequence of, for example, SEQ ID NO: 3.

The present inventors searched the genome of *Physcomitrium patens* for homologs of PPR-DYW, to identify PPR protein, which contains the GRP (Gly-Arg-Pro) domain instead of the DYW domain. The GRP (E2-GRP) domain, linked to the E2 domain, is an enzyme that catalyzes the reaction of transferring an amino group to uridine, to generate cytidine as described in Example 1 below. The amino acid sequence of the E2-GRP domain is, for example, shown in SEQ ID NO: 1 or SEQ ID NO: 2. As long as the enzyme has the activity that converts uridine to cytidine, the enzyme may be an E2-GRP-like protein of a plant other than *Physcomitrium patens,* may be an enzyme prepared by modifying cytidine deaminase to have the transamination activity, or may be an enzyme prepared by modifying an enzyme that transfers an amino group to free uridine, such that the enzyme acts on uridine in RNA.

The enzyme according to the present embodiment may be an enzyme having the activity of converting uridine in RNA to cytidine (hereinafter also referred to as "uridine-cytidine conversion activity"), and containing the following region (i) or (ii):
(i) a region having the amino acid sequence of SEQ ID NO: 1 or SEQ ID NO: 2; or
(ii) a region having an amino acid sequence having a sequence identity of 90% or more to the amino acid sequence of SEQ ID NO: 1 or SEQ ID NO: 2.

The sequence identity in (ii) is, for example, 91% or more, 92% or more, 93% or more, 94% or more, 95% or more, 96% or more, 97% or more, 98% or more, or 99% or more.

The enzyme according to the present embodiment may be a region having the uridine-cytidine conversion activity, and having the same amino acid sequence as SEQ ID NO: 1 or SEQ ID NO: 2 except that one or more amino acids are substituted, inserted, deleted, or added. The substituted, inserted, deleted, or added amino acids are one or several amino acids. The term "several" means, for example, not more than 20, not more than 15, or not more than 10. The term "several" preferably means any number within the range of 2 to 9.

The uridine-cytidine conversion activity of the enzyme according to the present embodiment can be evaluated by allowing *E. coli* or the like to express a target RNA containing uridine, and the enzyme, and then allowing the enzyme to act on the target RNA, followed by determining the base sequence of the target RNA. In cases where conversion of the uridine in the base sequence to cytidine occurs, the enzyme has the uridine-cytidine conversion activity. In order to allow the enzyme to act on the target RNA, a known sequence recognition module that allows the enzyme to act specifically on the uridine present in a specific region of the target RNA may be used. The sequence recognition module is described later.

Since the enzyme according to the present embodiment has the uridine-cytidine conversion activity, the enzyme can be used to convert uridine that has occurred in RNA due to mutation to cytidine.

In another embodiment, a polynucleotide encoding the enzyme is provided.

### Embodiment 2

A complex according to the present embodiment includes: the enzyme according to Embodiment 1; and a sequence recognition module that allows the enzyme to act specifically on uridine that has occurred in mRNA due to mutation. The uridine is, for example, uridine contained in a premature stop codon formed by nonsense mutation.

The sequence recognition module is not limited as long as the enzyme can be allowed to act on uridine present in a specific region of mRNA that serves as the target RNA. The sequence recognition module may be a single molecule, or a complex of a plurality of molecules. For example, the sequence recognition module contains at least one of a protein and a nucleic acid that bind to mRNA in a sequence-specific manner. More specific examples of the sequence recognition module include a CRISPR-dCAS system using dCas13 or the like whose nucleic acid cleavage ability has been deactivated, a zinc finger motif, a TAL effector, PPR protein, DNA, and peptide nucleic acid (PNA).

The CRISPR-dCas system uses a dCas protein whose nuclease activity and nickase activity have been lost, and a guide RNA. The guide RNA contains a base sequence that forms base pairs with the complementary strand of the target sequence, which corresponds to CRISPR RNA (crRNA), and a base sequence that functions as a trans-activating crRNA (tracrRNA) to serve as a scaffold for binding the dCas protein. Base pairing of the guide RNA with the complementary strand of the target sequence results in formation of a dCas-guide RNA complex. By using a fusion protein of the enzyme and the dCas protein, and a guide RNA designed for the vicinity of uridine that has occurred in mRNA due to mutation, the enzyme can be allowed to act specifically on the uridine.

A zinc finger motif contains a plurality of different zinc finger units of the Cys2His2 type linked to each other. Zinc finger motifs can be prepared by a known method such as the modular assembly method, OPEN method, CoDA method, or *E*. *coli* one-hybrid method. By using a fusion protein of a zinc finger motif designed for the vicinity of uridine that has occurred in mRNA due to mutation, and the enzyme, the enzyme can be allowed to act specifically on the uridine.

A TAL effector has a repeating structure of modules based on units of about 34 amino acids, and the 12th and 13th amino acid residues of each module determine the binding stability and the base specificity. For TAL effectors, the REAL method, the Golden Gate method, and the like have been established. By using a fusion protein of a TAL effector designed for the vicinity of uridine that has occurred in mRNA due to mutation, and the enzyme, the enzyme can be allowed to act specifically on the uridine.

PPR protein can be configured to recognize a specific base sequence by a sequence of PPR motifs each of which recognizes one nucleic acid base. By using a fusion protein of a PPR protein designed for the vicinity of uridine that has occurred in mRNA due to mutation, and the enzyme, the enzyme can be allowed to act specifically on the uridine.

In the backbone of PNA, units of N-(2-aminoethyl)glycine, which is not a sugar, are linked by amide bonds. In PNA, purine rings and pyrimidine rings corresponding to nucleic acid bases are bound to the backbone through methylene groups and carbonyl groups.

In the complex, the enzyme and the sequence recognition module may be linked to each other by a compound, linker, or the like through a covalent bond. The linker is a chemical group or a molecule that links the enzyme to the sequence recognition module. For example, the linker may link the enzyme to a protein or nucleic acid that binds to mRNA in a sequence-specific manner. The linker is positioned between two kinds of groups, molecules, or other portions, and bound to each of these by covalent bonding. The linker may be one amino acid or a plurality of amino acids. The linker may be an organic molecule, a group, a polymer, or the like. The linker has, for example, 3 to 200 amino acids. The linker may be a peptide of 3, 4, 5, 6, 7, 8, 9, 10, 10 to 15, 15 to 20, 30 to 35, 35 to 40, 40 to 45, 45 to 50, 50 to 60, 60 to 70, 70 to 80, 80 to 90, 90 to 100, 100 to 150, or 150 to 200 amino acids.

The following describes a case where the MS2 system is used as the sequence recognition module.

The genome of MS2 phage, which is an RNA virus, is a single-stranded RNA that functions as a plus-stranded mRNA. After MS2 phage infects *E. coli,* genes required for the growth of the MS2 phage are translated, and then a minus-stranded RNA is synthesized. The minus-stranded RNA is used as a template to synthesize a plus-stranded RNA. MS2 phage synthesizes various MS2 proteins based on the RNA. Among such proteins, MS2 coat protein binds upstream of the replicated gene.

In other words, in MS2 phage, the viral coat (outer shell) protein binds to the viral mRNA that has served as the translation template, that is, genomic RNA. By the MS2 system, a protein can be specifically linked to an RNA. In cases where the MS2 system is used, the sequence recognition module contains a guide RNA, an MS2 coat protein, and an MS2 RNA.

The guide RNA is an RNA complementary to at least part (target sequence) of mRNA containing uridine that has occurred in mRNA due to mutation. If the nucleic acid base U to be converted is regarded as "target nucleic acid base", the base sequence of the guide RNA may be constituted by a base sequence complementary to all bases in at least part of a base sequence containing the target nucleic acid base in mRNA, or the guide RNA may include a mismatch with the target sequence, that is, the base sequence of the guide RNA may be complementary to the bases of the target sequence except for one or several bases, as long as the guide RNA hybridizes with the target sequence. In cases where the guide RNA includes a mismatch, the mismatch is preferably limited to the nucleic acid base corresponding to the target nucleic acid base in the guide RNA. In such a case, the nucleic acid base corresponding to the target nucleic acid base in the guide RNA is a base other than adenine (A).

The guide RNA may form a fusion RNA with MS2 RNA (guide RNA - MS2 RNA). The fusion RNA can be prepared based on the base sequence of the guide RNA. For example, in the fusion RNA, the 3'-end or 5'-end of the guide RNA may be bound to one end of the MS2 RNA directly, or indirectly through a linker sequence or the like.

The enzyme may form a fusion protein with MS2 coat protein (MS2 coat protein - enzyme). The enzyme is bound to the guide RNA through binding of the MS2 coat protein to the MS2 RNA. For example, in the fusion protein, the C-terminus of the MS2 coat protein may be bound to the N-terminus of the enzyme directly, or indirectly through a linker peptide or the like. The fusion protein can be produced based on the gene sequences of the MS2 coat protein and the enzyme.

The MS2 RNA has a strong binding capacity to the MS2 coat protein. Therefore, by mixing the fusion protein and the fusion RNA together, or by allowing coexpression of the fusion protein and the fusion RNA in the cell, a complex including the guide RNA - the MS2 RNA - the MS2 protein - the enzyme can be obtained. The guide RNA allows the complex to bind complementarily to mRNA in the cell. For example, if the active site of the enzyme is guided to uridine that has occurred in mRNA due to mutation, the target nucleic acid base can be efficiently converted.

The sequence recognition module in the complex according to the present embodiment allows the enzyme that converts uridine to cytidine, to act specifically on the target uridine. Therefore, by targeting uridine that has occurred due to C > T point mutation, the mutant mRNA can be repaired. mRNA that has undergone nonsense mutation can be converted such that a full-length protein can be synthesized therefrom. Further, by allowing the complex according to the present embodiment to target uridine in a premature stop codon formed by nonsense mutation, RNA that has undergone nonsense mutation can be converted such that a full-length protein can be synthesized therefrom. As a result, the full-length protein can be produced.

In another embodiment, a polynucleotide encoding a complex including the fusion protein and the fusion RNA can be provided.

In cases where the sequence recognition module contains a nucleic acid that binds to mRNA in a sequence-specific manner, the nucleic acid may be DNA. The target sequence recognized by the nucleic acid does not necessarily need to contain the target nucleic acid base as long as the target sequence is in the vicinity of the target nucleic acid base. The target sequence in the vicinity herein may be, for example, a base sequence that starts at the 2nd to 10th, 2nd to 8th, 2nd to 6th, or 2nd to 4th base as counted from the target nucleic acid base in the direction toward the 5'-end of the mRNA, and that continues for a specific number of bases in the direction toward the 5'-end. Alternatively, the target sequence may be a base sequence that starts at the 2nd to 10th, 2nd to 8th, 2nd to 6th, or 2nd to 4th base as counted from the target nucleic acid base in the direction toward the 3'-end of the mRNA, and that continues for a specific number of bases in the direction toward the 3'-end. In cases where the sequence recognition module contains a protein that binds to mRNA in a sequence-specific manner, the base sequence in the mRNA recognized by the protein may be a base sequence that starts at the 2nd to 10th, 2nd to 8th, 2nd to 6th, or 2nd to 4th base as counted from the target nucleic acid base in the direction toward the 5'-end of the mRNA, and that continues for a specific number of bases in the direction toward the 5'-end. Alternatively, the base sequence in the mRNA recognized by the protein may be a base sequence that starts at the 2nd to 10th, 2nd to 8th, 2nd to 6th, or 2nd to 4th base as counted from the target nucleic acid base in the direction toward the 3'-end of the mRNA, and that continues for a specific number of bases in the direction toward the 3'-end.

A mutant of the MS2 coat protein may be used as long as the sequence-specific binding to the target mRNA can be achieved. Further, as the sequence recognition module, a known RNA-binding protein and an RNA in a system other than the MS2 system, such as the λN system may be used. The λN system uses the λN₂₂ peptide, and an RNA base sequence (Box-B) to which the λN₂₂ peptide specifically binds. A mutant of the λN₂₂ peptide may be used as long as the specific binding to the target mRNA can be achieved.

Examples of the RNA-binding protein include PP7 bacteriophage coat protein, Mu bacteriophage Com protein, stem-loop binding protein (SLBP), fragile X mental retardation syndrome-related protein 1 (FXR1), and proteins derived from bacteriophages, such as AP205, BZ13, f1, f2, fd, fr, ID2, JP34/GA, JP501, JP34, JP500, KU1, M11, M12, MX1, NL95, PP7, ΦCb5, ΦCb8r, ΦCb12r, ΦCb23r, Qβ, R17, SP-β, TW18, TW19, and VK; fragments thereof; and derivatives thereof.

### Embodiment 3

A recombinant vector according to the present embodiment allows intracellular expression and formation of the complex using the MS2 system according to Embodiment 2. More specifically, the recombinant vector allows intracellular expression and formation of a complex including the fusion protein and the fusion RNA in which the enzyme is bound to the guide RNA through binding of the MS2 coat protein to the MS2 RNA.

The recombinant vector may be a single recombinant vector including, based on the gene sequence of the enzyme, a first polynucleotide encoding the fusion protein of the enzyme and the MS2 protein, and a second polynucleotide encoding the guide RNA and the MS2 RNA, or may be separate recombinant vectors including the first polynucleotide and the second polynucleotide, respectively.

The base sequences of the first polynucleotide and the second polynucleotide can be determined based on the encoded amino acid sequences or on the RNA base sequences. The polynucleotides can be prepared based on the determined base sequences. The base sequences of the first polynucleotide and the second polynucleotide can be introduced into various vectors using restriction enzymes and DNA ligase, or using a plasmid construction kit based on homologous recombination.

The fusion protein may contain a linker sequence placed between the enzyme and the MS2 protein. There may also be a base sequence placed between the guide RNA and the MS2 RNA.

The recombinant vector according to the present embodiment enables expression of the fusion protein of the enzyme and the MS2 protein, and the MS2 RNA linked to the guide RNA. Thus, the enzyme that converts uridine to cytidine can be allowed to act specifically on the target nucleic acid base in mRNA in the cell. By the use of the recombinant vector, mutant mRNA can be repaired, or mRNA that has undergone nonsense mutation can be converted such that a full-length protein can be synthesized therefrom.

The recombinant vector enables expression and formation of the complex in the cell. By constituting the recombinant vector with DNA, which is more stable than RNA, the stability during storage and the like can be increased compared to the complex including the guide RNA, leading to greater ease of handling during the use.

### Embodiment 4

A genetic disease therapeutic agent according to the present embodiment includes the complex according to Embodiment 2, or the recombinant vector according to Embodiment 3. The genetic disease is a disease caused by nonsense mutation. The genetic disease may be a disease caused by T > C point mutation. Preferably, the genetic disease is a nonsense mutation-type genetic disease such as CF or DMD.

The genetic disease therapeutic agent according to the present embodiment can be produced by a known method. The genetic disease therapeutic agent may include not only the complex or recombinant vector as an effective component, but also other pharmaceutically acceptable components. The genetic disease therapeutic agent includes, for example, the complex or recombinant vector, and a pharmaceutically acceptable carrier. The pharmaceutically acceptable carrier is an organic carrier substance or inorganic carrier substance used as a pharmaceutical material. Examples of the pharmaceutically acceptable carrier include excipients, lubricants, binders, and disintegrators in solid formulations; and solvents, solubilizers, suspending agents, isotonic agents, buffers, and soothing agents in liquid formulations. The genetic disease therapeutic agent may also include, when necessary, an additive such as an antiseptic, an antioxidant, a coloring agent, or a sweetener.

The genetic disease therapeutic agent according to the present embodiment is administered to a human or an animal. The animal is preferably a mammal. Specific examples of the mammal include dogs, cats, cows, pigs, horses, sheep, and deer.

The dose of the genetic disease therapeutic agent according to the present embodiment is appropriately determined based on the sex, age, body weight, symptoms, and the like of the subject. The genetic disease therapeutic agent is administered such that an effective amount of the complex or recombinant vector is achieved. The effective amount is an amount of the complex or recombinant vector required for obtaining a desired result, and is an amount required for delaying, inhibiting, preventing, or reversing the progression of, or for curing of, the condition to be treated or handled.

The administration route of the genetic disease therapeutic agent for the human or the like is not limited. The genetic disease therapeutic agent is preferably used as an external preparation, an injection solution, or an orally administered agent.

The genetic disease therapeutic agent according to the present embodiment allows an enzyme that converts uridine to cytidine to act specifically on a target nucleic acid base in mRNA. By this, mRNA that has undergone nonsense mutation is converted such that a full-length protein can be synthesized therefrom, thereby enabling production of the full-length protein. Therefore, the genetic disease therapeutic agent is effective for treatment of a disease caused by nonsense mutation.

Another embodiment provides a method of treating, improving, or preventing a genetic disease in a subject by administering the complex according to Embodiment 2 or the recombinant vector according to Embodiment 3 to the subject. Another embodiment is use of the complex or recombinant vector for treatment, improvement, or prevention of a genetic disease. Another embodiment provides the complex or recombinant vector for use as a genetic disease therapeutic agent. Another embodiment is use of the complex or recombinant vector for the production of a genetic disease therapeutic agent. The complex according to Embodiment 2, or the recombinant vector according to Embodiment 3 may also be used as a reagent for, for example, an in vitro, in vivo, or ex vivo experiment.

### Examples

The present disclosure is described more concretely by way of the following Examples. However, the present disclosure is not limited by the Examples.

### Example 1: Detection of RNA Editing Activity of DYW Homolog Using PPR56

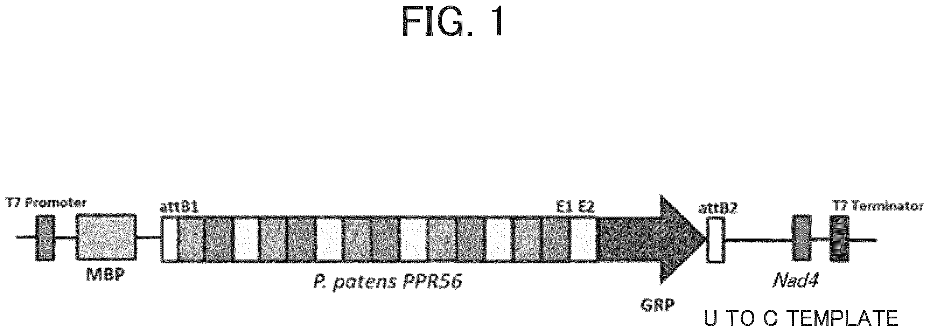

As illustrated in FIG. 1, a construct containing a GRP domain was prepared. The PPR56 gene of *Physcomitrium patens* (NCBI Accession Number: LOC112295756) was inserted downstream of the MPB promoter, and a GRP domain as a DYW homolog was inserted therein to replace the DYW domain of PPR56. Further, the *Nad4* gene as a target was inserted downstream. PPR56 binds to mRNA produced by transcription of the *Nad4* gene, to allow the GRP domain to approach the target nucleic acid base. A mutation was introduced to the corresponding position in the *Nad4* gene such that the target nucleic acid base was U. The base sequence of the *Nad4* gene to which the mutation was introduced is shown in SEQ ID NO: 4.

Using the construct prepared above, BL21, Rosetta 2 (DE3) was transformed by a conventional method, to obtain a bacterial strain. Two kinds of clones in which the GRP domain is inserted (GRP-g16507 (SEQ ID NO: 1) and GRP-g63 (SEQ ID NO: 2)) were obtained. Colonies were precultured in Luria broth with 50 µM kanamycin and 30 µM chloramphenicol. To 4 mL of the same medium, 40 µL of the preculture was inoculated, and culture was performed at 37°C until OD₆₀₀ became 0.4 to 0.6.

In the presence of 0.4 mM IPTG, 8 µL of 0.4 mM ZnSO₄ was added to induce gene expression. As a source of amino groups, 40 µL of 1 mM asparagine was added, and then culture was performed at 16°C at 180 rpm for 20 hours. After collecting 2 mL of a sample, mRNA was purified from the sample, and reverse transcription polymerase chain reaction (RT-PCR) was carried out. After the amplification, the base sequence was confirmed. The base sequences of the forward primer and the reverse primer used for the RT-PCR are respectively shown in SEQ ID NO: 5 and SEQ ID NO: 6.

### (Results)

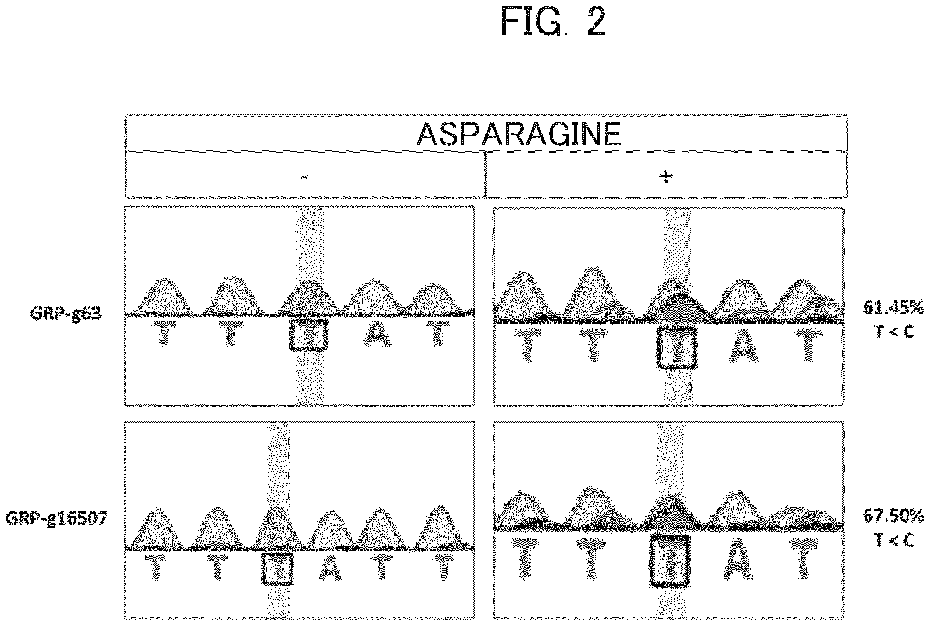

FIG. 2 illustrates part of the base sequence of DNA obtained as a result of the reverse transcription of the RNA to the DNA. For GRP-g63 and GRP-g16507, in the absence of asparagine, only a peak for thymine (T) could be detected at the position corresponding to the target nucleic acid base. However, in the presence of asparagine, a peak of cytosine (C) overlapping with a peak of thymine (T) could be detected for GRP-g63 and GRP-g16507 at 61.45% and 67.50%, respectively. The GRP domain was shown to have caused conversion of the target nucleic acid base U to C in part of the mRNA of the *Nad4* gene.

### Example 2: Detection of RNA Editing Activity of DYW Homolog Using PPR56

An experiment was carried out using HEK293 cells that show constitutive expression of a gene (BFP) obtained from the EGFP gene by a mutation from C to T that changes the fluorescence color from green to blue. In order to carry out deamination targeting the U in mRNA resulting from the mutation, a guide RNA having the base sequence complementary to a base sequence in the vicinity of the mutated base was prepared. The base sequence of the guide RNA is shown in SEQ ID NO: 7.

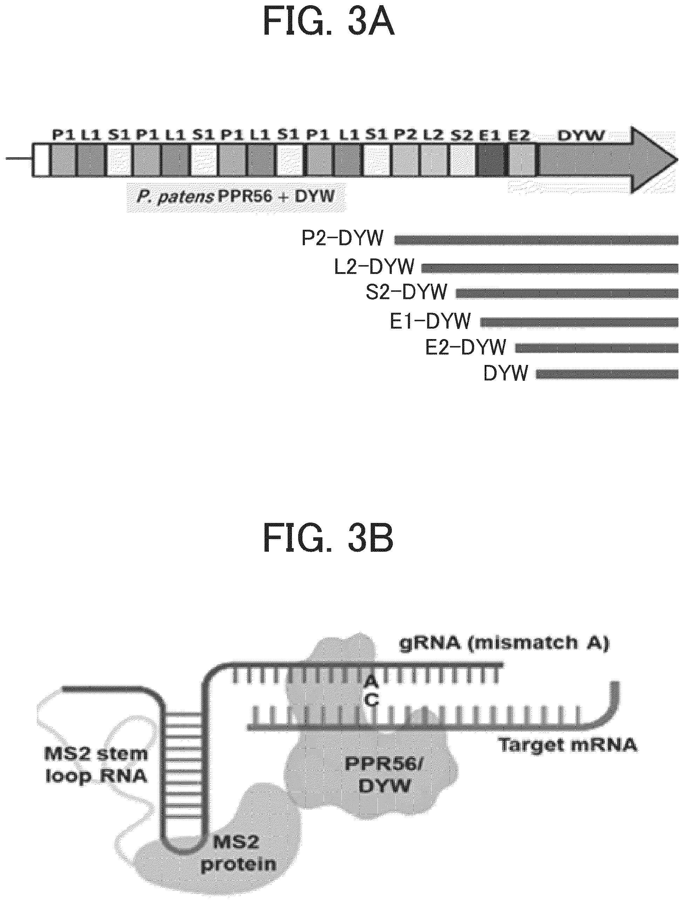

In order to confirm which site is indispensable for the RNA editing activity, defective proteins (P2-DYW, L2-DYW, S2-DYW, E1-DYW, E2-DYW, and DYW) lacking part of the upstream side of the PPR56 gene of *Physcomitrium patens* were studied as illustrated in FIG. 3A. A deaminase plasmid that expresses a gene encoding a fusion protein of a defective protein that replaces the PPR56/DYW illustrated in Fig. 3B, and MS2 coat protein was prepared. The amino acid sequences of P2-DYW, L2-DYW, S2-DYW, E1-DYW, E2-DYW, and DYW are respectively shown in SEQ ID NOs: 8, 9, 10, 11, 12, and 13.

The HEK293 cells stably expressing BFP were seeded at a density of 5.5 × 10⁵ cells/well. Before the transfection, the culture was removed such that the volume per well became 0.5 mL, and then 0.4 mL of fresh FB S-containing DMEM was added. Subsequently, 50 µL Opti-MEM was mixed with 500 ng of the plasmid (250 ng of the deaminase plasmid and 250 ng of the guide RNA), and the resulting mixture was incubated for 20 minutes at room temperature, followed by adding the mixture to each well. Green fluorescence was observed using a fluorescence microscope 48 hours after the transfection, and total RNA was extracted from the transfected cells. After reverse transcription of the extracted total RNA, sequence analysis was carried out to confirm the base sequence of the BFP gene or EGFP gene in the cells to which each defective protein gene was introduced.

### (Results)

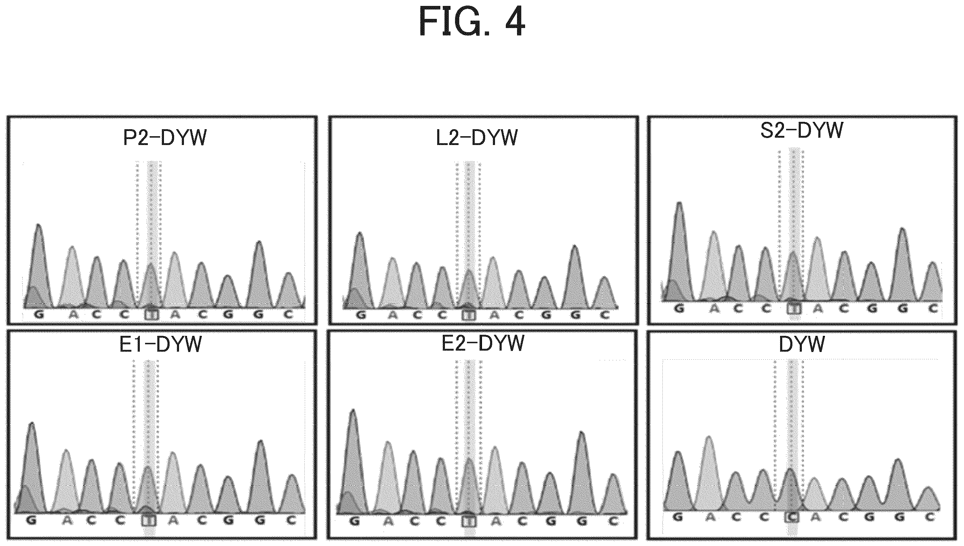

FIG. 4 illustrates the results of RNA editing by the introduction of the defective protein genes. The deamination activity of DYW required the E2 domain of the PPR protein. This was found to be true also for the GRP domain.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2021-210380, filed on December 24, 2021, the entire disclosure of which is incorporated by reference herein.

### Industrial Applicability

The present disclosure is useful for pharmaceuticals and research reagents.

## Claims

1. An enzyme having an activity that converts uridine in RNA to cytidine.

2. The enzyme according to claim 1, comprising:
(i) a region having an amino acid sequence of SEQ ID NO: 1 or SEQ ID NO: 2; or
(ii) a region having an amino acid sequence having a sequence identity of 90% or more to the amino acid sequence of SEQ ID NO: 1 or SEQ ID NO: 2.

3. A complex comprising:
the enzyme according to claim 1; and
a sequence recognition module that allows the enzyme to act specifically on uridine that has occurred in mRNA due to mutation.

4. The complex according to claim 3, wherein the uridine is uridine contained in a premature stop codon formed by nonsense mutation.

5. The complex according to claim 3 or 4, wherein
the sequence recognition module contains
a guide RNA complementary to at least part of a target RNA containing the uridine,
an RNA-binding coat protein derived from MS2 phage, and
an MS2 RNA that specifically binds to the RNA-binding coat protein,
the guide RNA forms a fusion RNA with the MS2 RNA, and
the enzyme forms a fusion protein with the RNA-binding coat protein, and is bound to the guide RNA through binding of the RNA-binding coat protein to the MS2 RNA.

6. A recombinant vector comprising:
a fusion protein of the enzyme according to claim 1 and an MS2 phage-derived RNA-binding coat protein; and
a fusion RNA of an MS2 RNA that specifically binds to the RNA-binding coat protein and a guide RNA complementary to at least part of a target RNA containing uridine that has occurred in mRNA due to mutation, wherein
the recombinant vector allows intracellular expression and formation of a complex in which the enzyme is bound to the guide RNA through binding of the RNA-binding coat protein to the MS2 RNA.

7. A genetic disease therapeutic agent comprising:
the complex according to claim 3; or
the recombinant vector according to claim 6.

8. A polynucleotide encoding the enzyme according to any one of claims 1 to 3.

9. A polynucleotide encoding a complex containing
a fusion protein of the enzyme according to claim 1 or 2 and an MS2 phage-derived RNA-binding coat protein; and
a fusion RNA of an MS2 RNA that specifically binds to the RNA-binding coat protein and a guide RNA complementary to at least part of a target RNA containing uridine that has occurred in mRNA due to mutation.
